# EUROPEAN PATENT SPECIFICATION

(11) **EP 2 995 301 B1**
(45) Date of publication and mention of the grant of the patent: **07.08.2019**
(21) Application number: 14794522.4
(22) Date of filing: 12.05.2014
(51) Int. Cl.: A61K 9/70, A61K 31/445, A61K 9/00

(54) **FILM PREPARATION CONTAINING DONEPEZIL-FREE BASE AND METHOD FOR PRODUCING SAME**
FILMHERSTELLUNG MIT DONEPEZIL ALS FREIE BASE UND VERFAHREN ZUR HERSTELLUNG DAVON
PRÉPARATION DE FILM CONTENANT UNE BASE LIBRE DE DONÉPÉZIL ET PROCÉDÉ DE PRODUCTION CORRESPONDANT

(30) Priority: 10.05.2013 KR 20130053355
(43) Date of publication of application: 16.03.2016
(73) Proprietor: CTC Bio, Inc., Seoul 138-858 (KR)
(72) Inventor: JEON, Hong-Ryeol, Suwon-si Gyeonggi-do 443-751 (KR); KWON, Do-Woo, Cheonan-si Chungcheongnam-do 331-760 (KR); LEE, Bong-Sang, Suwon-si Gyeonggi-do 443-757 (KR); PARK, Su-Jun, Yongin-si Gyeonggi-do 448-504 (KR); CHA, Bong-Geun, Hwaseong-si Gyeonggi-do 445-823 (KR); KIM, Jun-Ki, Cheongwon-gun Chungcheongbuk-do 363-844 (KR); HAN, Ji-Yeong, Ulsan 682-800 (KR); KIL, Myeong-Cheol, Hwaseong-si, Gyeonggi-do (KR)
(74) Representative: Nony
(86) International application number: PCT/KR2014/004218
(87) International publication number: WO 2014/182138

(56) References cited:
- WO-A1-2006/088337
- WO-A1-2010/146601
- WO-A1-2012/097197
- WO-A2-2007/125533
- WO-A2-2009/043588
- KR-A- 20090 033 000
- KR-B1- 101 239 150
- US-A1- 2008 131 491
- US-A1- 2010 173 940
- US-A1- 2011 237 563

## Description

### TECHNICAL FIELD

The present invention relates to a film formulation for oral cavity administration, comprising donepezil free base as an active ingredient, and a method for preparing the film formulation.

This application claims priority to Korean Patent Application No. 10-2013-0053355 filed in the Republic of Korea on May 10, 2013, which is incorporated herein by reference.

### BACKGROUND ART

Donepezil is a representative therapeutic agent among various commercially available agents for treating Alzheimer's disease, and it has been commercially available in the form of a tablet formulation containing donepezil hydrochloride.

Some patients that need therapeutic agents for Alzheimer's disease often reject taking drugs or are uncomfortable with swallowing or chewing the drugs. Accordingly, when having to use the therapeutic agents for Alzheimer's disease, in terms of transportability, it is preferable to use film forms, which are often called strips.

However, donepezil hydrochloride which has been commercially available is not easy to formulate in a film form, and thus a film formulation containing donepezil hydrochloride as an active ingredient is limited in its preparation for the following reasons:
Firstly, donepezil hydrochloride has its sharp and acrid taste, making it difficult to be formulated in a film form for oral cavity administration.
Secondly, in order to mask the taste of donepezil hydrochloride and expect the absorbance patterns (e.g., Cₘₐₓ, Tₘₐₓ) thereof, a considerable amount of an absorbance pattern-controlling agent and a considerable coating amount are required. From this, the weight of loading agents in a film becomes increased, or in order to load absorbance pattern-controlling agent in high amounts in a film having a limited size, the amount of donepezil hydrochloride and other additives is restricted, thereby failing to satisfy the desired activation effect, properties and handling of the film.

An orally administrable mucoadhesive film comprising a bioactive ingredient, such as donepezil, and an alginate as major film-forming polymer, has already been proposed in patent application No. WO 2007/125533 A2.

However, this document does not disclose a film containing a donepezil free base, even less in combination with a specific dispersing agent.

### DISCLOSURE

### Technical Problem

The present invention is designed to solve the above problems, and therefore it is an object of the present invention to provide a donepezil-containing film with no sharp taste and having a thickness and size suitable for administration as well as good handling and superior properties, and a method for preparing such a film.

### Technical Solution

In order to accomplish the object of the present invention, in accordance with one aspect of the present invention, there is provided a method for preparing a film comprising donepezil free base being tasteless as an active ingredient, and a film prepared by the method, more specifically a method for a donepezil free base-containing film for oral cavity administration, characterized by drying a polymer solution in which the free base of donepezil is dispersed (substantially not dissolved), wherein the polymer solution comprises a vinyl polymer-based compound selected from a group consisting of polyvinyl alcohol or polyvinylalcohol-based copolymers, polyvinyl acetate or polyvinyl acetate-based copolymers, polyvinyl pyrrolidone or polyvinyl pyrrolidone-based copolymers, or mixtures thereof as a dispersing agent for assisting the dispersion of the donepezil free base and said vinyl polymer-based compound being present in an amount of 0.1 to 50 wt% based on the total weight of the dried film, and a film prepared by the method.

The commercially available donepezil hydrochloride is not suitable for preparing a film formulation for oral cavity administration because it exhibits a particular sharp and acrid taste in oral cavity. Accordingly, the present inventors have endeavored to develop a new form of donepezil suitable in a film formulation and found that donepezil free base is tasteless in oral cavity to be suitable in the preparation of a film formulation.

Also, the present inventors have found that the donepezil hydrochloride is not suitable to be dispersed (suspended) due to its dissolution in water, whereas the donepezil free base is not almost dissolved in water to accomplish the desired object of the present invention.

In addition, the present inventors have found that the donepezil free base is suspended (dispersed) without the substantial dissolution thereof in a polymer solution as defined above to allow the formation of a film comprising the desired amount of donepezil and having a thickness and size suitable for administration as well as the desired properties.

Particularly, the present inventors have found that a vinyl polymer-based compound selected from a group consisting of polyvinyl alcohol or polyvinylalcohol-based copolymers, polyvinyl acetate or polyvinyl acetate-based copolymers, polyvinyl pyrrolidone or polyvinyl pyrrolidone-based copolymers, or mixtures thereof can be used to solve the problems of layer separation or non-homogenization caused by the water-insolubility of the donepezil free base, thereby providing good dispersion stability.

In the present invention, the film may be called a strip, orally dissolving film or orally disintegrating film, and refers to a formulation administered by attaching and dissolving the film on top and below the tongue, oral mucosa and in the mouth. The film formulation according to the present invention has an advantage in that it can be administered without water.

As used herein, the term "suspended without the substantial dissolution" means that donepezil free base is dissolved in an amount of 15 wt% or less, preferably 10 wt%, more preferably 7 wt%, still more preferably 4 wt%, most preferably 2 wt%, based on the total used weight thereof.

The donepezil free base may be present in an amount of 5 to 30 wt% based on the total weight of the dried film. If the amount of the donepezil free base is less than 5 wt%, it is uneconomical and incurs a problem during the preparation process. If the amount of the donepezil free base is higher than 30 wt%, the strength of the film may be lowered.

In the film formulation according to the present invention, the donepezil free base is not substantially dissolved, which restricts the interaction of the donepezil free base and a polymer used for forming a film. From this, it is expected that the formed film exhibits the desired properties even though the film comprises a high amount of the donepezil free base, but the present invention is not limited thereto.

In the case of donepezil, its particles may exhibit poor dispersion or reaggregated after dispersion owing to its insolubility. Therefore, the film formulation according to the present invention needs a dispersing agent that allows the uniform dispersion of the donepezil free base in the film, without substantially dissolving the donepezil free base.

The dispersing agent which used to disperse the donepezil free base in the present invention includes a vinyl polymer-based compound consisting of polyvinyl alcohol or polyvinylalcohol-based copolymers, polyvinyl acetate or polyvinyl acetate-based copolymers, polyvinyl pyrrolidone or polyvinyl pyrrolidone-based copolymers, or mixtures thereof. The addition of the vinyl polymer can maximize dispersion stability in the film without dissolving the donepezil free base and can inhibit the reaggregation of particles after a film solution is obtained.

The term "a vinyl polymer-based compound" refers to a group of polymers obtained by the addition polymerization of monomers having a vinyl group (-CH=CH₂), and have the same meaning as the term 'vinyl polymers" unless otherwise defined herein.

Examples of vinyl polymer-based compounds include polyvinyl alcohols (PVA), copolymers of polyethylene glycol/polyvinyl alcohol, polyvinyl pyrrolidone, polyvinyl acetate, physical combinations of polyvinyl pyrrolidone/polyvinyl acetate, copolymers of vinylpyrrolidone/vinylacetate, and graft copolymers of polycaprolactam-polyvinyl acetate-polyethylene glycol. More preferably, polyvinyl alcohol or polyvinylalcohol-based copolymers, polyvinyl acetate or polyvinyl acetate-based copolymers, polyvinyl pyrrolidone or polyvinyl pyrrolidone-based copolymers, or mixtures thereof may be used as a dispersing agent in the present invention.

In the present invention, the dispersing agent is present in an amount of 0.1 to 50 wt%, preferably 5 to 20 wt% based on the total weight of the dried film. If the amount of the dispersing agent is less than 0.1 wt%, the reaggregation of donepezil particles occurs in the prepared film, making it difficult for donepezil to be uniformly dispersed. If the amount of the dispersing agent is higher than 50 wt%, it is uneconomical, provides a particular taste and flavor, and incurs a problem during the preparation process.

In the present invention, the vinyl polymer-based compound is used for dispersion stabilization of donepezil. The use of the vinyl polymer-based compound as a dispersing agent allows more stable dispersion of the donepezil free base between the polymer chain as compared to a simply suspended dispersion solution of the donepezil free base, and can lower the amount of other additives used to reduce the aggregation of donepezil particles. For example, the total content of a dispersing agent, a plasticizer and a surfactant to be used can be lowered. Particularly, the amount of non-ionic surfactants (e.g., polysorbate 80) and anionic surfactants (e.g., sodium lauryl sulfate) can be lowered, thereby overcoming the problem that these surfactants dissolve a part of donepezil particles to exhibit a particular sharp taste.

Besides the vinyl polymer-based compound, examples of the dispersing agent, the plasticizer, and the surfactant which may be used in the film include docusate sodium, sodium lauryl sulfate, polysorbates, polyethylene glycol, propylene glycol, polyoxyethylene alkyl ethers, polyoxyethylene castor oil, polyoxyethylene stearate, triethyl citrate, glycerin and a mixture thereof, but are not limited thereto. Any other compounds known in the art may also be used unless deteriorating the object of the present invention.

The amount of vinyl polymer-based compound is 0.2 to 100 wt%, preferably 5 to 50 wt%, based on the total weight of the dispersing agent, the plasticizer and the surfactant present in the dried film.

In the present invention, it is preferred that a solvent used in a film-making solution comprises water in an amount of 90 wt% or more, preferably 95 wt% or more, more preferably 98 wt% or more thereof so that the donepezil free base is prevented from being dissolved in the film-making solution. Considering various aspects including a film thickness, a drying rate, a viscosity of a film-making solution in the coating of the film-making solution, the amount of a solvent used in the preparation of a film is preferably 0.7 to 4 parts by weight, more preferably 1.3 to 3.3 parts by weight, relative to 1 part by weight of other film components remained after drying.

More preferably, the present invention provides a method for preparing a donepezil free base-containing film for oral cavity administration, comprising dispersing donepezil free base in a solution obtained dissolving a polymer in a solvent comprising 90 wt% or more of water, and drying the solution having donepezil free base dispersed therein, to form a dried film having the polymer in an amount of 20 to 80 wt% based on the total weight of the dried film.

The polymer which may be used for forming the film in the present invention include pullulan, hydroxypropyl cellulose (HPC), hydroxypropylmethyl cellulose (HPMC), polyvinyl pyrrolidone (PVP), starch and a mixture thereof. Among these, considering the object of the present invention, pullulan and/or hydroxypropyl cellulose (HPC) are most preferred, in terms of the compatibility with the donepezil free base used as an active ingredient.

The film-making solution according to the present invention may further comprise a sweeting agent, a flavoring agent, or a colorant.

Further, the present invention provides a donepezil free base-containing film for oral cavity administration, prepared by the above-mentioned method characterized by drying a polymer solution in which donepezil free base as an active ingredient is dispersed, wherein the film comprises a vinyl polymer-based compound selected from a group consisting of polyvinyl alcohol or polyvinylalcohol-based copolymers, polyvinyl acetate or polyvinyl acetate-based copolymers, polyvinyl pyrrolidone or polyvinyl pyrrolidone-based copolymers, or mixtures thereof as a dispersing agent for assisting the dispersion of the donepezil free base, said vinyl polymer-based compound being present in an amount of 0.1 to 50 wt% based on the total weight of the dried film.

In particular, the film is prepared by drying the film-making solution according to the present invention which comprises a vinyl polymer-based selected from a group consisting of polyvinyl alcohol or polyvinylalcohol-based copolymers, polyvinyl acetate or polyvinyl acetate-based copolymers, polyvinyl pyrrolidone or polyvinyl pyrrolidone-based copolymers, or mixtures thereof being a dispersing agent and a polymer dissolved in a solvent comprising 90 wt% or more of water, and the donepezil free base dispersed therein, wherein the vinyl polymer-based compound is present in an amount of 0.2 to 100 wt% based on the total weight of a surfactant, a plasticizer and a dispersing agent. More preferably, the film is prepared by drying the solution in which 5 to 30 wt% of donepezil free base is dispersed and 0.1 to 50 wt% of a dispersing agent is used based on the total weight of the dried film, the film having the polymer in an amount of 20 to 80 wt% based on the total weight of the dried film. Also, in the film according to the present invention which comprises the vinyl polymer-based dispersing agent, the total weight of a plasticizer, a surfactant and/or a dispersing agent may ranges from 5 to 51 wt%. According to one embodiment, the film of the present invention further comprises a plasticizer, a surfactant and/or a dispersing agent, in addition to the vinyl polymer-based dispersing agent, and the amount of the vinyl polymer-based dispersing agent ranges from 0.2 to 100 wt% based on the total weight of the plasticizer, the surfactant, and the dispersing agent.

### Advantageous Effects

According to the film preparation method of the present invention, the present invention provides the film comprising the tasteless donepezil free base, wherein the donepezil free base uniformly is dispersed therein and the film has no sharp taste, a suitable thickness and size, as well as flexibility providing good handling stability and is not prone to breaking. The present invention also provides a donepezil free base-containing film for oral cavity administration prepared from the method.

### BEST MODE

Hereinafter, various preferred examples of the present invention will be described in detail for better understanding. However, the examples of the present invention may be modified in various ways, and they should not be interpreted as limiting the scope of the invention. The examples of the present invention are just for better understanding of the invention to persons having ordinary skill in the art.

### <Preparation of Donepezil Free Base-containing Film >

A donepezil free base-containing film formulation was prepared as follows. A plasticizer, an additive, a sweeting agent, a surfactant and a dispersing agent were added to purified water, followed by dissolving or dispersing by stirring, to which a donepezil free base was added. Then, homogenization was carried out using a homogenizer (Ultra turrax T-25, IKA). Thereto, a polymer was added and again homogenized using the same homogenizer to obtain a polymer solution having the donepezil free base dispersed therein. To the polymer solution, a flavoring agent was added and mixed by stirring. Then, the resulting film preparation was subject to degassing under vacuum, cooled to room temperature, and then coated on a PE film in a suitable thickness. The coating was dried at 80 °C to obtain a donepezil free base-containing film formulation.

### <Examples 1 to 16: Comparison of dispersion stabilization depending on the kinds of a dispersing agent to be added>

The procedures of the above "Preparation of Donepezil Free Base-containing Film" were repeated except that components and their content listed in Table 1 were used to prepare films. The prepared films were analyzed for the amount of the donepezil free base therein to measure dispersion stabilization. The results thereof are shown by relative standard deviation (RSD, %).

The RSD (%) for the amount of the donepezil free base was measured by cutting bulk films prepared into samples having a certain size and area. Then, in order to confirm a uniform dispersion degree of the donepezil free base, each sample obtained was analyzed for the amount of the donepezil free base present therein.

**Table 1**

| | Examples | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| Donepezil | 2.54 | 2.19 | 2.36 | 2.75 | 2.75 | 2.75 | 2.75 | 2.75 | 2.75 | 2.75 | 2.75 | 2.75 | 2.75 | 2.75 | 2.75 | 2.75 |
| Pullulan | 12.53 | 14.39 | 13.47 | 9.04 | 9.04 | 9.04 | 9.04 | 9.04 | 9.04 | 9.04 | 9.04 | 9.04 | 9.04 | 9.04 | 9.04 | 9.04 |
| Hydroxypropyl cellulose | 3.34 | 3.6 | 3.11 | 4.52 | 4.52 | 4.52 | 4.52 | 4.52 | 4.52 | 4.52 | 4.52 | 4.52 | 4.52 | 4.52 | 4.52 | 4.52 |
| Polyvinyl alcohol | | | | 2.45 | | | | | | | | | | | | |
| Hydroxyethyl cellulose | | | | | 2.45 | | | | | | | | | | | |
| Hydroxyethylm ethyl cellulose | | | | | | 2.45 | | | | | | | | | | |
| Copolymer of polycaprolactam -polyvinyl acetate-polyethylene glycol | | | | | | | 2.45 | | | | | | | | | |
| Copolymer of polyethylene glycol/polyvinyl alcohol | | | | | | | | 2.45 | | | | | | | | |
| Polyvinyl pyrrolidone (povidone) | | | | | | | | | 2.45 | | | | | | | |
| Polyvinyl acetate | | | | | | | | | | 2.45 | | | | | | |
| Copolymer of vinylpyrrolidone /vinylacetate | | | | | | | | | | | 2.45 | | | | | |
| Polyvinyl pyrrolidone/poly vinyl acetate(physical bonding) | | | | | | | | | | | | 2.45 | | | | |
| Polyoxyethylene castor oil | | | | | | | | | | | | | 2.45 | | | |
| Polyoxyethylene stearate | | | | | | | | | | | | | | 2.45 | | |
| Triethyl citrate | | | | | | | | | | | | | | | 2.45 | |
| Polyoxyethylene alkyl ether | | | | | | | | | | | | | | | | 2.45 |
| Glycerin | 4.46 | 3.84 | 4.15 | 4.82 | 4.82 | 4.82 | 4.82 | 4.82 | 4.82 | 4.82 | 4.82 | 4.82 | 4.82 | 4.82 | 4.82 | 4.82 |
| Titanium oxide | 0.56 | 0.48 | 0.52 | 0.6 | 0.6 | 0.6 | 0.6 | 0.6 | 0.6 | 0.6 | 0.6 | 0.6 | 0.6 | 0.6 | 0.6 | 0.6 |
| Sucralose | 0.97 | 0.84 | 0.91 | 1.05 | 1.05 | 1.05 | 1.05 | 1.05 | 1.05 | 1.05 | 1.05 | 1.05 | 1.05 | 1.05 | 1.05 | 1.05 |
| Flavor | Proper amount | Proper amount | Proper amount | Proper amount | Proper amount | Proper amount | Proper amount | Proper amount | Proper amount | Proper amount | Proper amount | Proper amount | Proper amount | Proper amount | Proper amount | Proper amount |
| RSD(%) of Content | 3.9 | 4.5 | 4.3 | 0.4 | 3.8 | 3.9 | 0.8 | 0.8 | 0.9 | 1.2 | 0.9 | 1.5 | 3.5 | 3.8 | 3.6 | 3.5 |
| Solvent | to 100% | | | | | | | | | | | | | | | |

As shown in Table 1, the use of a vinyl polymer-based compound as a dispersing agent provided good dispersion stabilization. Particularly, when polyvinyl alcohol was used as a dispersing agent (Example 4), the best result, i.e., the value of RSD(%) was 0.4. Also, the use of a vinyl polymer-based compound exhibited totally 1.5% or less of RSD, which provides substantially good dispersion stabilization as compared with the cases having no dispersing agent (Examples 1 to 3) and the cases having a dispersing agent other than the vinyl polymer-based compound.

Thus, the vinyl polymer-based compound can be used as a dispersing agent to allow the donepezil free base to be stably dispersed, thereby providing good stability.

## Claims

1. A donepezil free base-containing film for oral cavity administration, prepared by drying a polymer solution in which donepezil free base as an active ingredient is dispersed,
wherein the film comprises a vinyl polymer-based compound selected from a group consisting of polyvinyl alcohol or polyvinylalcohol-based copolymers, polyvinyl acetate or polyvinyl acetate-based copolymers, polyvinyl pyrrolidone or polyvinyl pyrrolidone-based copolymers, or mixtures thereof as a dispersing agent for assisting the dispersion of the donepezil free base, said vinyl polymer-based compound being present in an amount of 0.1 to 50 wt% based on the total weight of the dried film.

2. The film according to claim 1, wherein a solvent used in the polymer solution comprises water in an amount of 90 wt% or more thereof so that the donepezil free base is prevented from being dissolved.

3. The film according to claim 1 or 2, wherein the polymer used for forming the film is selected from the group consisting of pullulan, hydroxypropyl cellulose and mixture thereof.

4. The film according to any one of claims 1 to 3, which further comprises a plasticizer, a surfactant and/or a dispersing agent in an amount of 5 to 51 wt% based on the total weight of the dried film.

5. The film according to anyone of the claims 1 to 4, wherein the vinyl polymer-based compound is present in an amount of 0.2 to 100 wt% based on the total weight of the plasticizer, the surfactant, and the dispersing agent in the dried film.

6. A method for preparing a donepezil free base-containing film for oral cavity administration, comprising drying a polymer solution in which donepezil free base as an active ingredient is dispersed,
wherein the polymer solution comprises a vinyl polymer-based compound selected from a group consisting of polyvinyl alcohol or polyvinylalcohol-based copolymers, polyvinyl acetate or polyvinyl acetate-based copolymers, polyvinyl pyrrolidone or polyvinyl pyrrolidone-based copolymers, or mixtures thereof as a dispersing agent for assisting the dispersion of the donepezil free base and said vinyl polymer-based compound being present in an amount of 0.1 to 50 wt% based on the total weight of the dried film.

7. A method for preparing a donepezil free base-containing film for oral cavity administration, comprising drying a solution in which a vinyl polymer-based compound selected from a group consisting of polyvinyl alcohol or polyvinylalcohol-based copolymers, polyvinyl acetate or polyvinyl acetate-based copolymers, polyvinyl pyrrolidone or polyvinyl pyrrolidone-based copolymers, or mixtures thereof being a dispersing agent, and a polymer are dissolved in a solvent comprising 90 wt% or more of water, and the donepezil free base is dispersed,
wherein the vinyl polymer-based compound is present in an amount of 0.2 to 100 wt% based on the total weight of a surfactant, a plasticizer and a dispersing agent.

## Patentansprüche

1. Film, der die freie Base von Donepezil enthält, zur Verabreichung an die Mundhöhle, hergestellt durch Trocknen einer Polymerlösung, in der die freie Base von Donepezil als Wirkstoff dispergiert ist,
wobei der Film eine auf Vinylpolymer basierende Verbindung aus der Gruppe bestehend aus Polyvinylalkohol oder auf Polyvinylalkohol basierenden Copolymeren, Polyvinylacetat oder auf Polyvinylacetat, Polyvinylpurolidon oder auf Polyvinylpyrrolidon basierenden Copolymer basierenden Copolymeren oder Mischungen davon als Dispergiermittel zur Unterstützung der Dispergierung der freien Base von Donepezil umfasst, wobei die auf Vinylpolymer basierende Verbindung in einer Menge von 0,1 bis 50 Gew.-%, bezogen auf das Gesamtgewicht des getrockneten Films, vorliegt.

2. Film nach Anspruch 1, wobei das in der Polymerlösung verwendete Lösungsmittel Wasser in einer Menge von 90 Gew.-% oder mehr davon umfasst, so dass die freie Base von Donepezil an der Auflösung gehindert wird.

3. Film nach Anspruch 1 oder 2, wobei das zur Bildung des Films verwendete Polymer aus der Gruppe bestehend aus Pullulan, Hydroxypropylcellulose und Mischungen davon ausgewählt ist.

4. Film nach einem der Ansprüche 1 bis 3, der ferner einen Weichmacher, ein Tensid und/oder ein Dispergiermittel in einer Menge von 5 bis 51 Gew.-%, bezogen auf das Gesamtgewicht des getrockneten Films, umfasst.

5. Film nach einem der Ansprüche 1 bis 4, wobei die auf Vinylpolymer basierende Verbindung in einer Menge von 0,2 bis 100 Gew.-%, bezogen auf das Gesamtgewicht des Weichmachers, des Tensids und des Dispergiermittels in dem getrockneten Film, vorliegt.

6. Verfahren zur Herstellung eines Films, der die freie Base von Donepezil enthält, zur Verabreichung an die Mundhöhle, bei dem man eine Polymerlösung, in der die freie Base von Donepezil als Wirkstoff dispergiert ist, trocknet,
wobei die Polymerlösung eine auf Vinylpolymer basierende Verbindung aus der Gruppe bestehend aus Polyvinylalkohol oder auf Polyvinylalkohol basierenden Copolymeren, Polyvinylacetat oder auf Polyvinylacetat basierenden Copolymeren , Polyvinylpurolidon oder auf Polyvinylpyrrolidon basierenden Copolymer oder Mischungen davon als Dispergiermittel zur Unterstützung der Dispergierung der freien Base von Donepezil umfasst, wobei die auf Vinylpolymer basierende Verbindung in einer Menge von 0,1 bis 50 Gew.-%, bezogen auf das Gesamtgewicht des getrockneten Films, vorliegt.

7. Verfahren zur Herstellung eines Films, der die freie Base von Donepezil enthält, zur Verabreichung an die Mundhöhle, bei dem man eine Lösung, in der eine auf Vinylpolymer basierende Verbindung aus der Gruppe bestehend aus Polyvinylalkohol oder auf Polyvinylalkohol basierenden Copolymeren, Polyvinylacetat oder auf Polyvinylacetat basierenden Copolymeren oder Mischungen davon als Dispergiermittel und ein Polymer in einem Lösungsmittel, das 90 Gew.-% oder mehr Wasser umfasst, gelöst sind und die freie Base von Donepezil dispergiert ist, trocknet,
wobei die auf Vinylpolymer basierende Verbindung in einer Menge von 0,2 bis 100 Gew.-%, bezogen auf das Gesamtgewicht eines Tensids, eines Weichmachers und eines Dispergiermittels, vorliegt.

## Revendications

1. Film contenant du donézépil sous forme de base libre pour une administration par la cavité buccale, préparé par le séchage d'une solution de polymère dans laquelle du donézépil sous forme de base libre, en tant qu'ingrédient actif, est dispersé,
le film comprenant un composé à base de polymère vinylique choisi dans un groupe constitué par le poly(alcool vinylique) ou les copolymères à base de poly(alcool vinylique), le poly(acétate de vinyle) ou les copolymères à base de poly(acétate de vinyle), la polyvinylpyrrolidone ou les copolymères à base de polyvinylpyrrolidone ou les mélanges correspondants en tant qu'agent dispersant pour aider à la dispersion du donézépil sous forme de base libre, ledit composé à base de polymère vinylique étant présent en une quantité de 0,1 à 50 % en poids sur la base du poids total du film séché.

2. Film selon la revendication 1, dans lequel un solvant utilisé dans la solution de polymère comprend de l'eau en une quantité supérieure ou égale à 90 % de son poids, de sorte que le donézépil sous forme de base libre est empêché d'être dissout.

3. Film selon la revendication 1 ou 2, dans lequel le polymère utilisé pour la formation du film est choisi dans le groupe constitué par le pullulane, l'hydroxypropylcellulose et un mélange correspondant.

4. Film selon l'une quelconque des revendications 1 à 3, qui comprend en outre un plastifiant, un tensioactif et/ou un agent dispersant en une quantité allant de 5 à 51 % en poids sur la base du poids total du film séché.

5. Film selon l'une quelconque des revendications 1 à 4, dans lequel le composé à base de polymère vinylique est présent en une quantité allant de 0,2 à 100 % en poids sur la base du poids total du plastifiant, du tensioactif et de l'agent dispersant dans le film séché.

6. Procédé pour la préparation d'un film contenant du donézépil sous forme de base libre pour une administration par la cavité buccale, comprenant le séchage d'une solution de polymère dans laquelle du donézépil sous forme de base libre, en tant qu'ingrédient actif, est dispersé,
la solution de polymère comprenant un composé à base de polymère vinylique choisi dans un groupe constitué par le poly(alcool vinylique) ou les copolymères à base de poly(alcool vinylique), le poly(acétate de vinyle) ou les copolymères à base de poly(acétate de vinyle), la polyvinylpyrrolidone ou les copolymères à base de polyvinylpyrrolidone ou les mélanges correspondants en tant qu'un agent dispersant pour aider à la dispersion du donézépil sous forme de base libre et ledit composé à base de polymère vinylique étant présent en une quantité allant de 0,1 à 50 % en poids sur la base du poids total du film séché.

7. Procédé pour la préparation d'un film contenant du donézépil sous forme de base libre pour une administration par la cavité buccale, comprenant le séchage d'une solution dans laquelle un composé à base de polymère vinylique choisi dans un groupe constitué par le poly(alcool vinylique) ou les copolymères à base de poly(alcool vinylique), le poly(acétate de vinyle) ou les copolymères à base de poly(acétate de vinyle), la polyvinylpyrrolidone ou les copolymères à base de polyvinylpyrrolidone ou les mélanges correspondants, en tant qu'agent dispersant et un polymère sont dissouts dans un solvant comprenant 90 % en poids ou plus d'eau, et le donézépil sous forme de base libre est dispersé,
le composé à base de polymère vinylique étant présent en une quantité allant de 0,2 à 100 % en poids sur la base du poids total d'un tensioactif, d'un plastifiant et d'un agent dispersant.
